(19) Europäisches Patentamt
European Patent Office
Office européen des brevets

(11) **EP 4 202 846 A1**

(12) **EUROPEAN PATENT APPLICATION**

(43) Date of publication:
**28.06.2023 Bulletin 2023/26**

(51) International Patent Classification (IPC):
***G06T 17/20*** (2006.01)

(21) Application number: **21217220.9**

(22) Date of filing: **23.12.2021**

(52) Cooperative Patent Classification (CPC):
**G06T 17/20; A61B 5/00; G06T 19/20;**
G06T 2210/41; G06T 2219/2021

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **PAPINI, Ilan Meir**
**Eindhoven (NL)**
• **IBRAGIMOV, Zalman**
**Eindhoven (NL)**
• **GLUHOVSKY, Leonid**
**Eindhoven (NL)**

(74) Representative: **Philips Intellectual Property &
Standards
High Tech Campus 52
5656 AG Eindhoven (NL)**

(54) **ADAPTIVE 3D MODELLING OF AN ANATOMICAL CAVITY**

(57)    A mechanism for modifying a 3D mesh representing an anatomical cavity. The 3D mesh is defined within a co-ordinate system by tracking the location of any field-sensitive sensors carried by an interventional device. A new location is compared to the 3D mesh to determine whether it falls outside the bounds of the 3D mesh. If the new location falls outside the bounds, at least one vertex of the 3D mesh is modified based on the new location.

FIG. 2

EP 4 202 846 A1

**Description**

FIELD OF THE INVENTION

The present invention relates to the field of 3D modelling, and in particular to 3D modelling of anatomical cavities.

BACKGROUND OF THE INVENTION

There is an increasing interest in the (computer) modelling of anatomical cavities of a subject, e.g. to provide a visual representation of the anatomical element. This aids analysis of the anatomical element, e.g. during the course of a medical procedure or diagnosis.

One area of particular interest is in mapping of an anatomical element (such as the heart), in which an interventional device is inserted into the anatomical cavity of the anatomical element and one or more navigation and mapping systems are used to localize the ablation and measurement devices and/or sensors thereof. One example, of a suitable mapping system is the EPD Solutions navigation system provided by Philips®, named the KODEX-EPD® system, which exploits (di)electric field sensing for 3D localization, allowing radiation-free navigation in an anatomical cavity, e.g. inside the heart.

Some mapping techniques, such as those employed by the KODEX-EPD system, are based on electrical fields between three pairs of electrode-patches that represent the x-, y-, and z-dimension. During the procedure, voltages induced at one or more field-sensitive sensors carried by the interventional device are monitored, and a real-time voltage-to-position function is formed. This function is continuously updated based on the measurements coming in. Once sufficiently updated, the voltage-to-position function enables the system to display the spatial movement of the device within a predefined co-ordinate system reliably.

The stored measurements are used to anatomically reconstruct an anatomical model of the anatomical cavity (e.g. inside the heart). The position of the device can then be determined with respect to the anatomical model, allowing accurate navigation towards and around anatomical features.

There is a clear desire for reducing inaccuracies of mapping, i.e. to provide a more accurate anatomical model of the anatomical cavity. This will aid in assessment and/or diagnosis of the subject, as well as improving an accuracy of monitoring the true location of an interventional device within the anatomical cavity.

SUMMARY OF THE INVENTION

The invention is defined by the claims.

According to examples in accordance with an aspect of the invention, there is provided a computer-implemented method for modifying an anatomical model of an anatomical cavity.

The computer-implemented method comprises: obtaining the anatomical model of the anatomical cavity, wherein: the anatomical model was generated by monitoring the location, within a predefined co-ordinate system, of at least one field-sensitive sensor of an interventional device as the interventional device was moved within the anatomical cavity; and the anatomical model comprises a 3D mesh defined by a plurality of vertices, each vertex being associated with a different location in the predefined co-ordinate system; obtaining a new location, within the predefined co-ordinate system, that was obtained by a field-sensitive sensor of the interventional device within the anatomical cavity; determining whether or not the new location falls outside the bounds of the 3D mesh of the anatomical model; responsive only to the new location being predicted to fall outside the bounds of the 3D mesh of the anatomical model, modifying the location of one or more of the vertices of the 3D mesh based on the new location; and optionally outputting, if the locations of one or more of the vertices of the 3D mesh are modified, the 3D mesh with the one or more modified vertices.

Embodiments provide a mechanism for modifying a 3D mesh representing the bounds of an anatomical cavity when new location information is made available. The 3D mesh may be a 3D surface mesh. The proposed approach avoids the need to reconstruct or reassemble a completely new 3D mesh when a new location for the (at least one) field sensitive sensor is moved within the anatomical cavity, whilst still providing a suitably accurate 3D mesh representation of the anatomical cavity.

Embodiments are based on the realization that the 3D mesh is only likely to be inaccurate if a new location is outside of the bounds of the 3D mesh. By only modifying the 3D mesh if the new location is outside of the bounds of the 3D mesh (i.e. not on or in the 3D mesh), then the 3D mesh can be accurately updated to correctly represent the true bounds of the anatomical cavity. The proposed approach provides a "painting-in" type construction of the 3D mesh.

The proposed approach can be performed in between iterative updates of the 3D mesh, e.g. between iterative generations of a 3D mesh from monitored locations.

In some examples, the new location is predicted to fall outside the bounds of the 3D mesh only when the new location does not lie within the 3D mesh and a distance between the new location and the nearest location on the bounds of the anatomical model exceeds a first predetermined non-zero threshold.

In some examples, the new location is predicted to fall outside the bounds of the 3D mesh only when the new location does not lie within the 3D mesh and a distance between the new location and the location of the nearest vertex of the anatomical model exceeds a second predetermined non-zero threshold.

These two approaches provide a margin of error for determining whether or not a new location falls outside the bounds of the 3D mesh. This avoids potentially unnecessary modifications to the 3D mesh. This approach also accounts for any minor errors in determining the new location, e.g. due to inhomogeneity of fields used to define or determine the new location.

In at least one example, the step of determining whether or not the new location falls outside the bounds of the 3D mesh of the 3D mesh comprises: identifying, as a closest vertex, the vertex of the 3D mesh associated with the location closest to the new location; determining a first direction vector, being the direction vector from the location of the closest vertex to the new location; identifying, as a first normal, a normal to the 3D mesh at the closest vertex; determining a dot product between the first vector and the first normal; and determining that the new location falls outside the bounds of the 3D mesh of the anatomical model when the value of the dot product exceeds a predetermined dot product threshold.

The step of modifying the location of one or more of the vertices of the 3D mesh may comprise: identifying, as a closest vertex, the vertex of the 3D mesh associated with the location closest to the new location; and modifying the location associated with the closest vertex responsive to a distance between the new location and the location associated with the closest vertex.

In at least one example, the modifying the location associated with the closest vertex comprises setting the modified location of the closest vertex to lie at a location falling part way between the location of the closest vertex and the new location.

The step of modifying the location of one or more of the vertices of the 3D mesh may comprise: identifying, as proximate vertices, any vertices of the 3D mesh associated with a location that fall within a predetermined distance of the new location or the location associated with the closest vertex; and for each proximate vertex, modifying the location associated with the proximate vertex based on the new location.

In some examples, modifying the location associated with the closest vertex comprises setting the modified location of the closest vertex to lie at the new location.

In some examples, for each proximate vertex, the modifying of the location associated with the proximate vertex is responsive to a direction vector starting at the location associated with the closest vertex and terminating at the new location.

In particular, the direction vector or a weighted version of the direction vector may be added or applied to the location associated with the proximate vector to produce the modified location for the proximate vector.

In some examples, for each proximate vertex, the modifying of the location associated with the proximate vertex is further responsive to the distance between the location associated with the proximate vertex and the location associated with the closest vertex.

In particular examples, the direction vector is weighted (for the proximate vertex) using the distance between the location associated with the proximate vertex and the location associated with the closest vertex. In particularly preferable examples, the greater the distance, the lower the weighting of the direction vector. This would produce a smoother transition or change to the 3D mesh.

In some embodiments, for each proximate vertex, modifying the location associated with the proximate vertex is responsive to the reciprocal of the distance between the location associated with the proximate vertex and the location associated with the closest vertex.

In at least one embodiment, for each proximate vertex, modifying the location associated with the proximate vertex is responsive to a distance difference divided by the predetermined distance, wherein the distance difference is the difference between the predetermined distance and the distance between the location associated with the proximate vertex and the location associated with the closest vertex.

In some examples, for each proximate vertex, modifying the location associated with the proximate vertex comprises multiplying the direction vector by a weighting value, the weighting value being equal to the distance difference divided by the predetermined distance to produce a weighted direction vector; and adding the weighted direction vector to the location associated with the proximate vertex to modify the location associated with the proximate vertex.

The method may further comprise, if one or more of the vertices of the 3D mesh are modified based on the new location, providing a visual representation of the 3D mesh with the one or more modified vertices at a user interface.

There is also proposed a computer program product comprising computer program code means which, when executed on a computing device having a processing system, cause the processing system to perform all of the steps of any herein described method.

There is also proposed a processing system for modifying an anatomical model of an anatomical cavity.

The processing system comprises an input interface configured to: obtain the anatomical model of the anatomical cavity, wherein: the anatomical model was generated by monitoring the location, within a predefined co-ordinate system, of at least one field-sensitive sensor of an interventional device as the interventional device moves within the anatomical cavity; and the anatomical model comprises a 3D mesh defined by a plurality of vertices, each vertex being associated with a different location in the predefined co-ordinate system; and obtain a new location, within the predefined co-ordinate system, that was obtained by a field-sensitive sensor of the interventional device within the anatomical cavity;

The processing system also comprises a processing unit configured to: predict whether or not the new location falls outside the bounds of the 3D mesh of the anatomical model; and responsive only to the new location being predicted to fall outside the bounds of the 3D mesh of the anatomical model, modify the location of one or more of the vertices of the 3D mesh based on the new location.

The processing system may comprise an output unit configured to, if the locations of one or more of the vertices of the 3D mesh are modified, output the 3D mesh with the one or more modified vertices.

Any herein described processing system may be appropriately configured to carry out any herein described method, and vice versa.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates a system for analyzing an anatomical cavity of a subject;
Figure 2 conceptually illustrates an approach for converting a point cloud into a 3D mesh for an anatomical model
Figure 3 illustrates a method according to an embodiment;
Figure 4 illustrates an example step for use in an embodiment;
Figure 5 illustrates another example step for use in an embodiment;
Figure 6 illustrates a processing system according to an embodiment;
Figure 7 also illustrates a processing system according to an embodiment; and
Figure 8 illustrates a system according to an embodiment.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for modifying a 3D mesh representing an anatomical cavity. The 3D mesh is defined within a co-ordinate system by tracking the location of any field-sensitive sensors carried by an interventional device. A new location is compared to the 3D mesh to determine whether it falls outside the bounds of the 3D mesh. If the new location falls outside the bounds, at least one vertex of the 3D mesh is modified based on the new location.

Embodiments are based on the realization that new location that lies outside the bounds of the 3D mesh indicates that the 3D mesh does not accurately represent the shape of the anatomical cavity. Modifying one or more vertices based on such a new location facilitates prompt and efficient updating of the 3D mesh, without needing to reconstruct the 3D mesh from a cloud of points. Embodiments thereby provide a more efficient mechanism for providing a 3D mesh that accurately represents the bounds of the anatomical cavity.

Proposed concepts may be employed during or after a medical procedure to update an anatomical model of an anatomical cavity. This would provide a clinician with useful information for assessing the condition of the subject.

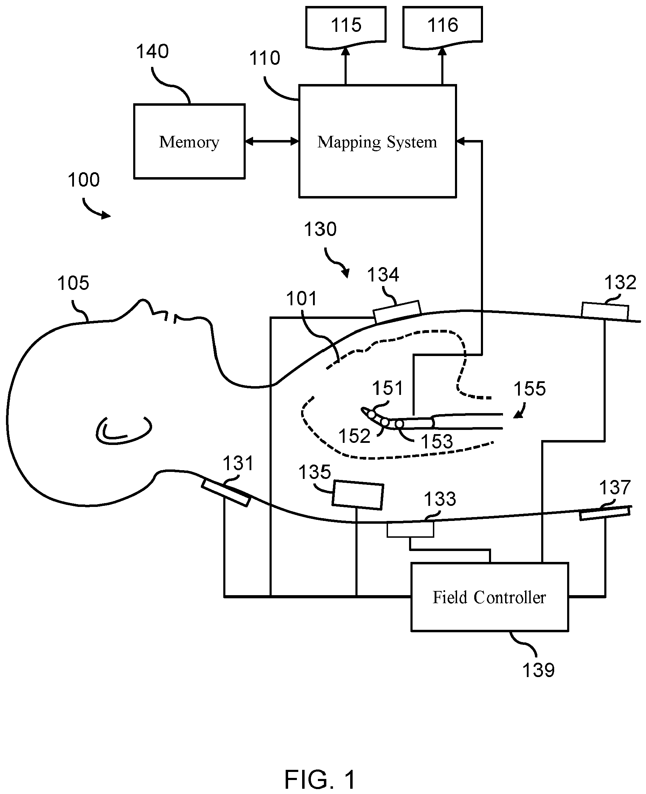

Figure 1 conceptually illustrates a system 100 for analyzing an anatomical cavity 101 of a subject 105. The anatomical cavity may, for instance, be an anatomical cavity within any suitable anatomical element of the subject, such as the heart, liver, kidney, lungs etc.

The system 100 comprises a mapping and navigation system 110 configured to generate an anatomical model 115 of the anatomical cavity 101. The anatomical model is generated by monitoring the location of at least one field-sensitive sensor 151, 152, 153 of an interventional device 155 as the interventional device was moved within the anatomical cavity. The interventional device 155 may be a catheter, although other examples will be apparent to the skilled person.

Generally, construction of an anatomical model is performed by first generating a cloud of points within a defined space, each point representing a position or location of the interventional device (and/or a sensor electrode on the interventional device) within a defined space. Thus, each point may be defined by a set of co-ordinates or the like. This cloud of points is subsequently processed to construct an anatomical model.

One approach for monitoring the location of a field sensitive sensor as the interventional device moves within the anatomical cavity is to use a magnetic sensor that responds to crossing magnetic fields induced within the subject. In other words, the at least one field-sensitive sensor 151, 152, 153 may each comprise a magnetic sensor. Different magnetic responses of the magnetic sensor(s) represent different locations of the magnetic sensor(s) in a predefined co-ordinate space. In this way, as the interventional device is moved within the anatomical cavity, a collection or cloud of (location) points can be constructed from the magnetic responses of the magnetic sensor.

Approaches for monitoring the location using a magnetic sensor are established in the art, for instance as described by, inter alia, WO 2017/201288 A1, US 2021/052191 A1 and WO 2012/150567 A1. Any of these approaches, or others established in the art, could be employed for use in the present disclosure.

Another approach for monitoring the location of a field-sensitive sensor as the interventional device moves within the anatomical cavity is to monitor the electrical response of one or more electrodes of the interventional device ("internal electrodes") to crossing electric fields induced within the subject. In other words, the at least one field-sensitive sensor 151, 152, 153 may each comprise an electrode that responds to changes in electric field strength. This approach is illustrated by Figure 1.

An electric field generating arrangement 130 may be configured to generate these crossing electrical fields using a set of external electrodes 131, 132, 133, 134, 135, 137, which are positioned externally with respect to the subject 105. This is performed by a field controller 139 appropriately controlling characteristics (e.g. voltage and/or current) of the electric signals provided to each external electrode.

The crossing electric fields may be controlled to have a different frequency with respect to one another, which effectively facilitates triangulation of the electrode within an electric field space.

The set 130 of external electrodes may comprise a plurality of electrode pairs angled with respect to one another (e.g. orthogonal to one another), so that any electric fields generated by the electrode pairs are angled with respect to one another. These electrode pairs may comprise a first electrode pair (formed of a first 131 and second 132 external electrode), a second electrode pair (formed of a third 133 and fourth 134 external electrode) and a third electrode pair (formed of a fifth 135 and sixth (not visible) external electrode). One of more of these electrode pairs may be omitted. The set of external electrodes may also comprise a reference electrode 137.

The electric field generator 139 may be configured to control the electric fields to operate in the frequency range of 30-100 kHz. This frequency range is particularly useful for ensuring penetration of a subject, whilst providing a frequency range that attenuates in human tissue without significant damage/injury to the tissue. The reference electrode serves as an electric reference for all measurements taken by electrodes, e.g. as a reference for the response of the internal electrodes.

The electrical response of an internal electrode 151, 152, 153, e.g. the voltage present at an internal electrode, to the electric fields changes as the relative position of the internal electrode moves about the subject. This is at least partly because the induced electrical fields' distribution is inherently inhomogeneous due to the different dielectric properties and absorption rates (related to conductivity) of the interrogated tissues. The principle of crossing electric fields thereby facilitates tracking of the relative position of the interventional device using a mapping system 110 that monitors a response of any internal electrodes to the crossing electric fields, e.g. by mapping the (electrical) response of an electrode to a relative position within the subject or using a suitable mapping/transfer function, e.g. the "V2R function", to determine the relative position(s) of the electrodes, and therefore the interventional device, in a predefined co-ordinate space. This a predefined co-ordinate space is sometimes called an R-space.

Approaches for generating such a mapping function are well established in the art. Example processes are disclosed by the European Patent Applications EP 0775466 A2, EP 3568068 A1 and EP 3607879 A1. Typically, generating a mapping function makes use of known interelectrode distances to calibrate for an inhomogeneity in the electric fields.

Once a number of locations of the interventional device and/or one or more sensors/electrodes on the interventional device in the predefined co-ordinate system have been established, it is possible to construct an anatomical model 115 of the anatomical cavity 101 (i.e. a cavity in which the interventional device is able to move). This process may also be performed by the mapping system 110.

Broadly, the mapping system 110 may build an anatomical model of an anatomical cavity 101 (e.g. a chamber, vessel or void) within a subject by processing a cloud of points. Each point represents a location or position of the interventional device or sensor/electrode in the predefined co-ordinate space, and could therefore be represented as a set of data, e.g. a set of co-ordinates. The cloud of points may thereby be generated using any previously described process for monitoring locations.

More specifically, a reconstruction algorithm is used to generate an anatomical model of the (investigated part of the) anatomical cavity. The anatomical model may, for example, be a 3D mesh or surface that depicts or models the (known bounds of) the anatomical cavity. In the present disclosure, the anatomical model comprises a 3D mesh defined by a plurality of vertices, each vertex being associated with a different location in the predefined co-ordinate system. The 3D mesh is therefore defined in the same co-ordinate system as the determined locations of the field-sensitive sensor(s). The 3D mesh may, for instance, be defined as sets of co-ordinates defining vertices of the 3D mesh.

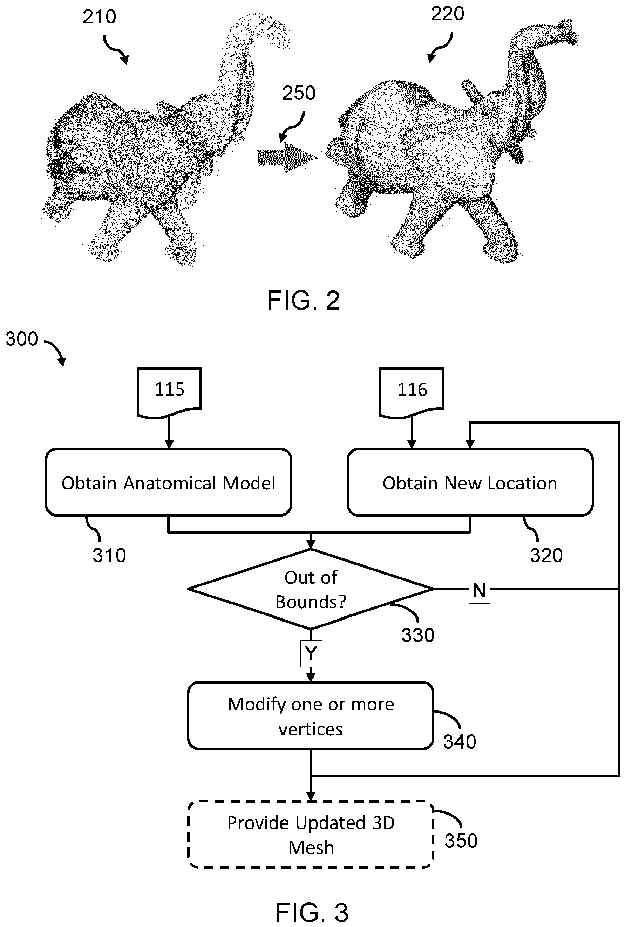

The process of reconstructing an anatomical model from a point cloud is conceptually illustrated in Figure 2, which demonstrates a process 250 in which a cloud of points 210 ("point cloud") is transformed into an anatomical model 220. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

The anatomical model may be stored, for instance, in a memory 140 or storage unit. Of course, the anatomical model may be output for further processing and/or to a user interface configured to provide a visual representation of the anatomical model.

More particularly, once a 3D mesh has been generated then a visual representation of the 3D mesh can be provided. Providing a visual representation of the 3D mesh may comprise, for example, rendering a reconstruction of the surface of the (known) anatomical cavity.

It will be apparent that once an anatomical model has been generated, it is possible to monitor the location of the field-sensitive sensor(s) and/or interventional device with respect to the anatomical model. The mapping system 110 may be accordingly configured to determine a location of the field-sensitive sensor(s) and/or the interventional device. This may comprise identifying a new location 116 of each field-sensitive sensor.

The monitored (new) location may be used, for instance, to provide a visual representation of the known bounds of the anatomical cavity (represented by the anatomical model) and the relative position of the interventional device with respect to the anatomical cavity.

It is also possible to map measured parameters of the anatomical cavity (or the anatomical element containing the anatomical cavity) to positions on the 3D mesh of the anatomical model. For instance, the interventional device may configured to obtain one or more electrophysiological (EP) parameters, such as impedance, and map each obtained EP parameter to location (by determining the location at which the EP parameter was sampled). In this way, an overlay of one or more EP parameters with respect to the 3D mesh may be determined or derived.

The present disclosure proposes a mechanism for modifying the 3D mesh of the anatomical model responsive to one or more new locations of a field-sensitive sensor. Thus, rather than completely reconstructing a new 3D mesh from an updated cloud of points, the 3D mesh is directly updated responsive to a determined new location. This process may be performed in between iterative updating and processing of the cloud of points to construct a new 3D mesh, in order to quickly provide updated 3D meshes in-between iterative reconstructions of the 3D mesh from updated point clouds.

In the context of the present disclosure, a location is considered to be a location in the predefined co-ordinate space. Thus, a location may be formed of data identifying a location in the predefined co-ordinate space (e.g. a set of co-ordinates).

Figure 3 illustrates a computer-implemented method 300 according to an embodiment of the invention. The computer-implemented method 300 may, for instance, be performed by the mapping system. Alternatively, method 300 may be performed by a further processing system.

The method 300 comprises a step 310 of obtaining the anatomical model 115 of the anatomical cavity. As previously explained, the anatomical model was generated by monitoring the location, within a predefined co-ordinate system, of at least one field-sensitive sensor of an interventional device as the interventional device was moved within the anatomical cavity.

The anatomical model comprises a 3D mesh defined by a plurality of vertices, each vertex being associated with a different location in the predefined co-ordinate system. The anatomical model comprises a dataset defining at least a 3D mesh. This dataset defines a location of a plurality of vertices (of the 3D mesh) within the predefined co-ordinate system. For instance, the dataset may comprise sets of co-ordinates, each set of co-ordinate representing a different vertex of the 3D mesh.

Step 310 may comprise, for instance, retrieving the anatomical model from the memory or storage unit. Alternatively, step 310 may be performed by using an anatomical model carried by the mapping system.

The method 300 also comprises a step 320 of obtaining a new location 116, within the predefined co-ordinate system, that was obtained by a field-sensitive sensor of the interventional device within the anatomical cavity. The new location represents a location for a field-sensitive sensor within the anatomical cavity that was not originally included in the cloud of (location) points used to generate the 3D mesh of the anatomical model. The new location may be formed of data identifying a location in the predefined co-ordinate space (e.g. a set of co-ordinates).

The method 300 then performs a step 330 of determining whether or not the new location falls outside the bounds of the 3D mesh of the anatomical model. Any location within the predefined co-ordinate system can be defined as falling inside the bounds of a 3D mesh (i.e. surrounded by the 3D mesh), on the bounds of the 3D mesh or outside the bounds of the 3D mesh (i.e. not surrounded by the 3D mesh).

For the sake of further explanation, it will be understood that the 3D mesh defines the bounds of a volume in the predefined co-ordinate system. A new location may be considered to fall outside the bounds of the 3D mesh if it does not lie within this volume or on the surface of this volume (i.e. on the 3D mesh itself).

Some embodiments may make use of an error margin when determining whether or not the new location falls outside the bounds of the 3D mesh. This accounts for any errors in the mapping of a response of a field-sensitive sensor to the pre-defined co-ordinate system. Thus, determining whether the new location falls outside the bounds of the 3D mesh may comprise determining whether the field-sensitive sensor is more than some predetermined distance outside the bounds of the 3D mesh. A more complete example of this approach will be provided later in this disclosure.

The method moves to step 340 only if the new location is predicted to fall outside the bounds of the 3D mesh. Otherwise, the method may revert to step 320 of obtaining a (further) new location. Of course, if no new location is available, the method may idle at step 320 until a (further) new location is made available.

Step 340 comprises modifying the location of one or more of the vertices of the 3D mesh based on the new location. In other words, one or more of the vertices may be displaced if the new location falls outside the bounds of the 3D mesh, as determined in step 330. Thus, the 3D mesh may effectively be warped or deformed if it is determined that the new location falls outside the (known) bounds of the anatomical cavity.

One approach for performing step 330 is to predict that the new location falls outside the bounds of the 3D mesh only when the new location does not lie within the 3D mesh and a distance between the new location and the nearest location on the bounds of the anatomical model exceeds a first predetermined non-zero threshold. Otherwise, the new location may be considered to fall within the bounds of the 3D mesh.

Another approach for performing step 330 is to predict that the new location falls outside the bounds of the 3D mesh only when the new location does not lie within the 3D mesh and a distance between the new location and the location of the nearest vertex of the anatomical model exceeds a second predetermined non-zero threshold. Otherwise, the new location may be considered to fall within the bounds of the 3D mesh.

Suitable values for the first and/or second predetermined non-zero threshold may be values that represent a distance of between 1 and 5mm, for example, 3mm.

Yet another approach for performing step 330 is to identify, as a closest vertex, the vertex of the 3D mesh associated with the location closest to the new location; determine a first direction vector, being the direction vector from the location of the closest vertex to the new location; identify, as a first normal, a normal to the 3D mesh at the closest vertex; determine a dot product between the first vector and the first normal; and predict that the new location falls outside the bounds of the 3D mesh of the anatomical model when the value of the dot product exceeds a predetermined dot product threshold. Otherwise, the new location may be considered to fall within the bounds of the 3D mesh.

Thus, step 330 may comprise the following steps: find the closest vertex V1 to the new location L1 and find the closest vertex's normal N1. Calculate the dot product of the vector between the closest vertex and the new location (the first distance vector) and the normal at the closest vertex. The normal N1 is a normal, e.g. a direction extending perpendicularly out of the 3D mesh, to the 3D mesh at the location of the closest vertex. This produces a distance measure D1 as follows:

$$D1 = (E1 - V1) * N1 \tag{1}$$

If the value of D1 is greater than some predetermined dot product threshold T1, then the new location is predicted to lie outside the bounds of the 3D mesh. The value of T1 may represent a distance of between 1 and 5mm, for example, 3mm (converted, if appropriate, to the scale and/or units of the predefined co-ordinate system).

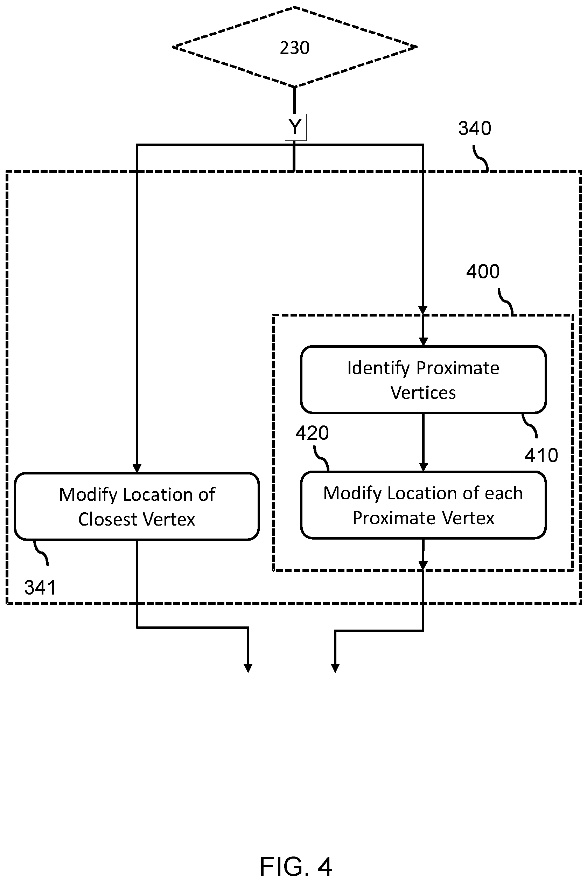

Figure 4 illustrates various embodiments for step 340 of method 300 illustrated in Figure 3.

In some examples, step 340 comprises a sub-step 341 of modifying the location of the closest vertex to the new location. The closest vertex is the vertex of the 3D mesh that is associated with the location closest to the new location. This approach effectively warps the position of the closest vertex based on the new location.

In some examples, sub-step 341 comprises setting the modified location of the closest vertex to lie at a location falling part way between the location of the closest vertex and the new location, e.g. at a location at a predetermined percentage along a path between the location of the closest vertex and the new location.

Alternatively, sub-step 341 may comprise setting the modified location of the closest vertex to lie at the new location. This approach may be particularly advantageous as it sets the modified location to a known new location, thereby providing a more accurate anatomical model.

Another example for performing sub-step 341 is provided later.

In some examples, step 340 comprises a process 400.

Process 400 comprises a step 410 of identifying, as proximate vertices, any vertices of the 3D mesh associated with a location that fall within a predetermined distance of the new location or the location associated with the closest vertex (as previously defined).

Step 410 may be performed, for instance, by processing each vertex of the 3D mesh to identify whether that vertex falls within a predetermined distance of the new location or the location associated with the closest vertex.

Process 400 also comprises a step 420 of, for each identified proximate vertex, modifying the location associated with the proximate vertex based on the new location.

Step 410 may comprise, for instance, identifying any other vertex (other than the closest vertex) of the 3D mesh for which a distance D2 between the location Vn of this vertex and the location V1 of the closest vertex is less than a predetermined distance T2 (i.e. any vertex for which D2 < T2, where D2 is the distance between Vn and VI). A generalized Pythagorean Theorem can be readily used to determine a distance between two locations in a predefined co-ordinate system. The value of the predetermined distance T2 may, for instance, be a distance of between 10mm and 30mm, for example, 20mm (converted, if appropriate, to the scale and/or units of the predefined co-ordinate system).

Step 420 may only be performed on those vertices identified in step 410, i.e. only on the proximate vertices.

In other examples, step 420 and sub-step 341 are merged into a single step. In particular, the closest vertex V1 may be treated as a proximate vertex and undergo the same procedure for modifying its position. Thus, step 420 may be performed on the proximate vertices and the closest vertex (which acts as a proximate vertex).

In step 420, the modifying of the location associated with the proximate vertex may be responsive to a difference or distance between the new location and the location associated with the closest vertex.

In some examples, for each proximate vertex, the modifying of the location associated with the proximate vertex is further responsive to the difference or distance between the location associated with the proximate vertex and the location associated with the closest vertex. For instance, the distance between the location of the proximate vertex and the location of the closest vertex may be used to weight a modification performed on the proximate vertex.

In some examples step 420 comprises, for each proximate vertex, modifying the location associated with the proximate vertex responsive to the reciprocal of the difference between the location associated with the proximate vertex and the location associated with the closest vertex.

A working example for step 420 is hereafter described. As previously mentioned, the same procedure can be used to perform step 341 (e.g. in which the closest vertex V1 also act as a proximate vertex Vn). Thus, steps 420 and 341 can be performed at a same time, i.e. using a same process.

For each proximate vertex Vn, a distance D2 between the location of the proximate vertex Vn and the location of the closest vertex V1 can be calculated. This distance may have been previously calculated (e.g. in step 410) to identify or select any proximate vertices, e.g. by comparison to a predetermined distance T2. If the closest vertex V1 is being treated as a proximate vertex, the distance D2 is equal to 0 for the closest vertex V1.

A weight Wn is then calculated, for weighting a modification to be performed on the proximate vertex. The weight Wn may be responsive to the distance or difference D2 between the location of the closest vertex V1 and the location of the proximate vertex. In particular, the value of Wn may decrease with increasing distance between the location of the proximate vertex and the location of the closest vertex.

In one example, the weight Wn is calculated as follows:

$$Wn = \frac{(T2 - D2)}{T2} \qquad (2)$$

The approach of equation (2) provides a simple and efficient mechanism for calculating a weight that varies with increasing distance from the closest vertex.

In another example, the weight Wn is calculated as follows:

$$Wn = \frac{1}{X + D2^M} \qquad (3)$$

where M is any positive value equal to or greater than 1, e.g. 1 or 2 and X is greater than or equal to 1. Setting X to be greater than or equal to 1 ensures that the value of Wn increases with increasing distance (i.e. the weight reduces with increasing distance). X may be omitted from equation (3) if it can be otherwise guaranteed that the value of Wn increases with increasing distance, e.g. if distance D2 is always greater than or equal to 1.

The approach of equation (3) provides an alternative mechanism for calculating a weight that varies with increasing distance from the closest vertex.

If either of equations (2) or (3) are used to determine the weight Wn, it will be appreciated that the value of the weight Wn will be equal to 1 if the proximate vertex Vn is the closest vertex V1. This is because the value of D2 will be 0 in these scenarios.

An offset or modification vector On may then be defined. The modification vector effectively defines the direction in which the location of the proximate vector is to be changed.

The offset or modification vector On may be defined as the direction vector from the location of the closest vertex V1 to the new location L1 (i.e. L1-V1). Thus, the offset or modification vector may be the same for all of the proximate locations.

Alternatively, the offset or modification vector On may be defined as the direction vector from the location of the proximate vertex Vn to the new location L1 (i.e. L1-Vn). Thus, the offset or modification vector may be different for all of the proximate locations.

The location of vertex Vn can then be updated to a new location Vn' using the following equation:

$$Vn' = Vn + Wn.On \tag{4}$$

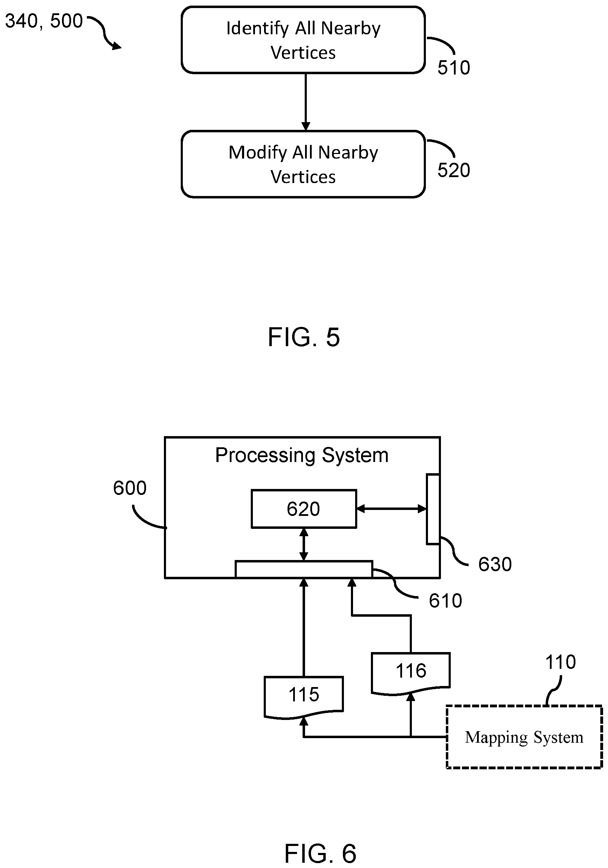

Figure 5 illustrates an alternative approach 500 to performing step 340 of method 300 described in Figure 3.

In this example, the approach 500 comprises a step 510 of identifying all vertices Vx of the 3D mesh having a location lying within a predetermined threshold distance TD of the new location L1. The closest vertex V1 will be one of the vertices Vx lying within the predetermined threshold distance TD of the new location L1. A distance between the location of the vertex Vx and the new location may be defined as D3.

The approach 500 may then comprise a step 520 of modifying, for each identified vertex Vx, a location of the vertex Vx based on a direction vector from the location of the identified vertex and the new location (i.e. L1-Vx). For instance, the modified location Vx' of each vertex Vx could be calculated as follows:

$$Vx' = Vx + Wx.(L1 - Vx) \tag{5}$$

Alternatively, step 520 may comprise modifying, for each identified vertex Vx, a location of the vertex Vx based on a direction vector from the location of the closest vertex V1 and the new location (i.e. L1-V1). For instance, the modified location Vx' of each vertex Vx could be calculated as follows:

$$Vx' = Vx + Wx.(L1 - V1) \tag{6}$$

The value of Wx is a weight to be applied to the modification. The value of Wx may be calculated as:

$$Wx = \frac{(TD - D3)}{TD} \tag{7}$$

As an alternative example, the value of Wx may be calculated as:

$$Wx = \frac{1}{X + D3^M} \tag{8}$$

where M is any positive value equal to or greater than 1, e.g. 1 or 2 and X is greater than or equal to 1. Setting X to be greater than or equal to 1 ensures that the value of Wx increases with increasing distance (i.e. the weight reduces with increasing distance).

In some examples, the value of Wx is set to 1 for the closest vertex. However, this is not essential.

In this way, the relative displacement of vertices that are closer to the new location is greater than those that are further away. This provides a graduated or smoother transition to the mesh.

Turning back to Figure 3, the method 300 may further comprise a step 350 of providing or outputting the 3D mesh, e.g. to a user interface and/or to a further processing system. In some examples, step 350 is only performed if one or more of the vertices of the 3D mesh are modified based on the new location.

Step 350 may, for instance, comprise outputting the anatomical model with the updated 3D mesh.

For the avoidance of doubt, it is noted that the anatomical model and/or 3D mesh is a dataset containing information that defines the 3D surface. The 3D mesh thereby represents data (representing a 3D surface) that can be processed by a data processor or the like.

In some examples, step 350 comprises providing a visual representation of the 3D mesh with the one or more modified vertices at a user interface. To provide a visual representation, step 350 may comprise rendering the visual representation of the 3D mesh. Rendering may comprise, for instance, rendering triangles connecting the vertices of the 3D mesh to provide a visual representation of the bounds of the anatomical cavity.

Steps 320-340 may be repeated for each of a plurality of different new locations. For instance, if an interventional device carries more than one field-sensitive sensor, each of which provides a separate new location, then steps 310-340 may be performed a plurality of times each time a new update on the samples taken by the field-sensitive sensor is available.

In some examples, the method 300 may be iteratively repeated (i.e. restart at step 310) each time a new (3D) mesh is available. As previously described, each new location may be added to the cloud of points from which a mesh is generated. A new mesh may therefore be iteratively generated using the updated cloud of points.

One advantage of the proposed mechanism is that updating or modifying vertices of an existing mesh can be performed more quickly and efficiently than generating an entirely new mesh from a cloud of points. This means that a more efficient approach for generating a mesh (e.g. for rendering and display to a user) can be exploited.

Thus, there is proposed a method of iteratively reconstructing or generating a 3D mesh using a cloud of points, wherein the cloud of points is iteratively updated or appended with each new location obtained from the field-sensitive sensor(s) of the interventional device. In this method, in between or during each iterative reconstruction of the 3D mesh, the most recently reconstructed/generated 3D mesh may be updated using any previously described method (and the new location(s) obtained from the field sensitive sensor(s)). The method for performing reconstruction is more resource intensive (and therefore is performed more slowly) than the proposed mechanism for updating a most recently reconstructed/generated 3D mesh.

Figure 6 illustrates a processing system 600 for modifying an anatomical model of an anatomical cavity. The processing system may form part of the mapping system illustrated in Figure 1, or may be a separate system configured to receive data generated by the mapping system.

The processing system 600 comprises an input interface 610 configured to obtain the anatomical model 115 of the anatomical cavity. The anatomical model is one that was generated by monitoring the location, within a predefined co-ordinate system, of at least one field-sensitive sensor of an interventional device as the interventional device moves within the anatomical cavity. The anatomical model comprises a 3D mesh defined by a plurality of vertices, each vertex being associated with a different location in the predefined co-ordinate system.

The input interface 610 is also configured to obtain a new location 116, within the predefined co-ordinate system, that was obtained by a field-sensitive sensor of the interventional device within the anatomical cavity.

The anatomical model 115 and/or the new location 116 may be obtained from the mapping system 110. Alternatively, the anatomical model and/or the new location may be obtained from a memory. Of course, a combination of these possibilities could be used, e.g. the anatomical model may be obtained from a memory and the new location may be obtained from the mapping system. When the processing system 600 is integrated into the mapping system, the anatomical model and/or the new location may be obtained from another module of the mapping system and/or a memory or storage unit.

The processing system 600 also comprises a processing unit 620 communicatively coupled to the input interface.

The processing unit is configured to predict whether or not the new location falls outside the bounds of the 3D mesh of the anatomical model; and responsive only to the new location being predicted to fall outside the bounds of the 3D mesh of the anatomical model, modify the location of one or more of the vertices of the 3D mesh based on the new location.

The processing system may be appropriately adapted to perform any herein described method, as would be readily apparent to the skilled person. For instance, the input interface may be appropriately adapted to perform any embodiment of steps 310 and 320 herein described, and the processing unit 620 may be appropriately adapted to perform any of steps 330 and/or 340 herein described.

The processing system may further comprise an output interface 630 configured to, if the locations of one or more of the vertices of the 3D mesh are modified, output the 3D mesh with the one or more modified vertices.

In some examples, the processing unit 620 is configured to control, e.g. via the output interface 630, a visual representation of the 3D mesh. This may comprise the processing unit comprise rendering a visual representation of the 3D mesh and controlling a user interface to provide the visual representation. Rendering may comprise, for instance, rendering triangles connecting the vertices of the 3D mesh to provide a visual representation of the bounds of the anatomical cavity.

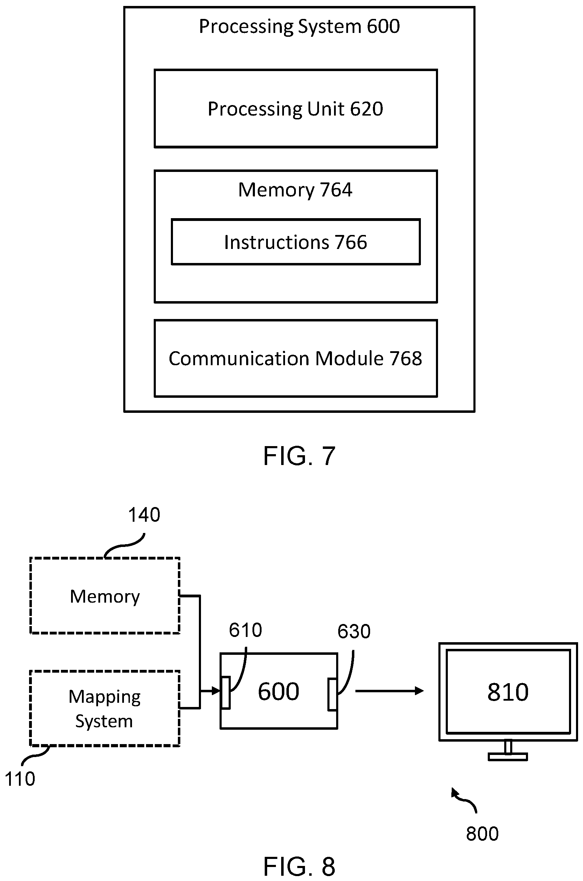

Figure 7 is a schematic diagram of a processing system 600, according to embodiments of the present disclosure.

The processing system 600 is configured to carry out a method according to an embodiment, such as the method 300 described with reference to Figure 3.

As shown, the processing system 600 may include a (data) processing unit 620, a memory 764, and a communication module 768. These elements may be in direct or indirect communication with each other, for example via one or more buses.

The processing unit 620 may include a central processing unit (CPU), a digital signal processor (DSP), an ASIC, a controller, an FPGA, another hardware device, a firmware device, or any combination thereof configured to perform the operations described herein. The processing unit 620 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 764 may include a cache memory (e.g., a cache memory of the processing unit 620), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 764 includes a non-transitory computer-readable medium. The non-transitory computer-readable medium may store instructions. For example, the memory 764, or non-transitory computer-readable medium may have program code recorded thereon, the program code including instructions for causing the processing system 600, or one or more components of the processing system 600, particularly the processing unit 620, to perform the operations described herein. For example, the processing system 600 can execute operations of the method 500.

Instructions 766 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 764, with the code recorded thereon, may be referred to as a computer program product.

The communication module 768 can include any electronic circuitry and/or logic circuitry to facilitate direct or indirect communication of data between the processing system 600, the penetration device and/or the user interface (or other further device). In that regard, the communication module 768 can be an input/output (I/O) device. In some instances, the communication module 768 facilitates direct or indirect communication between various elements of the processing system 600 and/or the processing arrangement (Figure 8).

Figure 8 illustrates a processor system 800 according to an embodiment of the invention, which illustrates some optional elements. The processor system 800 comprises a processing system 600, which may be embodied as previously described.

The processor system may further comprise an output display 810. The processing system may be configured to control the output display 810 to display a visual representation of any data generated by the processing system 600, such as any updated 3D meshes.

This control may be performed using an output interface 630 of the processing system 600. The visual representation of any 3D mesh may be generated by performing a rendering process on the 3D mesh.

The processing system 600 may be adapted to obtain the anatomical model and/or the new location from a memory 140 and/or systems that generate such models, such as the mapping system 110, e.g. at an input interface 610. The processor system 800 may comprise the memory and/or the systems as appropriate.

It will be appreciated that the mapping system 110 and the processing system 600 may thereby form aspects of a single processing system, e.g. each form different sub-systems or processes performed by the single processing system. In the context of the present disclosure, this means that a single system could perform the actions of a mapping system and a processing system as herein described.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored there on the computer program (product) previously described. There is also proposed a data carrier signal carrying the computer program (product) previously described.

The skilled person would be readily capable of developing a processing system for carrying out any herein described method. Thus, each step of a flow chart may represent a different action performed by a processing system, and may be performed by a respective module of the processing system.

It will be appreciated that different disclosed systems may form part of a single processing system, e.g. each disclosed system is a sub-system of a single processing system. In the context of the present disclosure, this means that a single system could perform the actions of a mapping system and a processing system as herein described.

An input/output interface may represent internal circuitry of a larger system, e.g. connected to an input/output of a processing unit. The same processing unit may be reused to perform different tasks, such as: generating the anatomical model and modifying the anatomical model (or 3D mesh).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

**Claims**

1. A computer-implemented method (300) for modifying an anatomical model (115) of an anatomical cavity (101), the computer-implemented method comprising:
   obtaining (310) the anatomical model of the anatomical cavity, wherein:

   the anatomical model was generated by monitoring the location, within a predefined co-ordinate system, of at least one field-sensitive sensor (151, 152, 153) of an interventional device (150) as the interventional device was moved within the anatomical cavity; and
   the anatomical model comprises a 3D mesh defined by a plurality of vertices; each vertex being associated with a different location in the predefined co-ordinate system;
   obtaining (320) a new location (116), within the predefined co-ordinate system, that was obtained by a field-sensitive sensor of the interventional device within the anatomical cavity;
   determining (330) whether or not the new location falls outside the bounds of the 3D mesh of the anatomical model;
   responsive only to the new location being predicted to fall outside the bounds of the 3D mesh of the anatomical model, modifying (340) the location of one or more of the vertices of the 3D mesh based on the new location; and
   optionally outputting (350), if the locations of one or more of the vertices of the 3D mesh are modified, the 3D mesh with the one or more modified vertices.

2. The computer-implemented method of claim 1, wherein the new location is predicted to fall outside the bounds of the 3D mesh only when the new location does not lie within the 3D mesh and a distance between the new location and the nearest location on the bounds of the anatomical model exceeds a first predetermined non-zero threshold.

3. The computer-implemented method of claim 1, wherein the new location is predicted to fall outside the bounds of the 3D mesh only when the new location does not lie within the 3D mesh and a distance between the new location and the location of the nearest vertex of the anatomical model exceeds a second predetermined non-zero threshold.

4. The computer-implemented method of claim 1, wherein determining whether or not the new location falls outside the bounds of the 3D mesh of the 3D mesh comprises:

identifying, as a closest vertex, the vertex of the 3D mesh associated with the location closest to the new location;

determining a first direction vector, being the direction vector from the location of the closest vertex to the new location;

identifying, as a first normal, a normal to the 3D mesh at the closest vertex;

determining a dot product between the first vector and the first normal; and

determining that the new location falls outside the bounds of the 3D mesh of the anatomical model when the value of the dot product exceeds a predetermined dot product threshold.

5. The computer-implemented method of any of claims 1 to 4, wherein the step of modifying the location of one or more of the vertices of the 3D mesh comprises:

identifying, as a closest vertex, the vertex of the 3D mesh associated with the location closest to the new location; and

modifying (341) the location associated with the closest vertex responsive to a distance between the new location and the location associated with the closest vertex.

6. The computer-implemented method of claim 5, wherein modifying the location associated with the closest vertex comprises setting the modified location of the closest vertex to lie at the new location.

7. The computer-implemented method of any of claims 5 or 6, wherein the step of modifying the location of one or more of the vertices of the 3D mesh comprises:

identifying (410), as proximate vertices, any vertices of the 3D mesh associated with a location that fall within a predetermined distance of the new location or the location associated with the closest vertex; and

for each proximate vertex, modifying (420) the location associated with the proximate vertex based on the new location.

8. The computer-implemented method of claim 7, wherein, for each proximate vertex, the modifying of the location associated with the proximate vertex is responsive to a direction vector starting at the location associated with the closest vertex and terminating at the new location.

9. The computer-implemented method of claim 8, wherein, for each proximate vertex, the modifying of the location associated with the proximate vertex is further responsive to the distance between the location associated with the proximate vertex and the location associated with the closest vertex.

10. The computer-implemented method of claim 9, wherein, for each proximate vertex, modifying the location associated with the proximate vertex is responsive to the reciprocal of the distance between the location associated with the proximate vertex and the location associated with the closest vertex.

11. The computer-implemented method of claim 9, wherein, for each proximate vertex, modifying the location associated with the proximate vertex is responsive to a distance difference divided by the predetermined distance, wherein the distance difference is the difference between the predetermined distance and the distance between the location associated with the proximate vertex and the location associated with the closest vertex.

12. The computer-implemented method of claim 11, wherein for each proximate vertex, modifying the location associated with the proximate vertex comprises:

multiplying the direction vector by a weighting value, the weighting value being equal to the distance difference divided by the predetermined distance to produce a weighted direction vector; and

adding the weighted direction vector to the location associated with the proximate vertex to modify the location associated with the proximate vertex.

13. The computer-implemented method of any of claims 1 to 12, further comprising, if one or more of the vertices of the 3D mesh are modified based on the new location, providing a visual representation of the 3D mesh with the one or more modified vertices at a user interface.

14. A computer program product comprising computer program code means which, when executed on a computing device having a processing system, cause the processing system to perform all of the steps of the method according to any of claims 1 to 13.

15. A processing system (600) for modifying an anatomical model (115) of an anatomical cavity (101), the processing system comprising:

an input interface (610) configured to:
obtain the anatomical model of the anatomical cavity, wherein:
the anatomical model was generated by monitoring the location, within a predefined co-ordinate system, of at least one field-sensitive sensor (151, 152, 153) of an interventional device (150) as the interventional device moves within the anatomical cavity; and
the anatomical model comprises a 3D mesh defined by a plurality of vertices, each vertex being associated with a different location in the predefined co-ordinate system; and
obtain (320) a new location (116), within the predefined co-ordinate system, that was obtained by a field-sensitive sensor of the interventional device within the anatomical cavity;
a processing unit (620) configured to:

predict (330) whether or not the new location falls outside the bounds of the 3D mesh of the anatomical model; and
responsive only to the new location being predicted to fall outside the bounds of the 3D mesh of the anatomical model, modify (340) the location of one or more of the vertices of the 3D mesh based on the new location; and
optionally, an output unit (630) configured to, if the locations of one or more of the vertices of the 3D mesh are modified, output (350) the 3D mesh with the one or more modified vertices.

FIG. 1

FIG. 2

FIG. 3

230

Y

340

400

Identify Proximate
Vertices

410

420

Modify Location of
Closest Vertex

Modify Location of each
Proximate Vertex

341

FIG. 4

340, 500

Identify All Nearby
Vertices ⟩ 510

↓

Modify All Nearby
Vertices ⟩ 520

## FIG. 5

Processing System

600

620 ↔ ▯ ⟩ 630

↕

▭ ⟩ 610

↑          ↑

115    116    110

Mapping System

## FIG. 6

Processing System 600

Processing Unit 620

Memory 764

Instructions 766

Communication Module 768

FIG. 7

140

Memory

110

Mapping System

610

630

600

810

800

FIG. 8

Europäisches
Patentamt
European
Patent Office
Office européen
des brevets

# EUROPEAN SEARCH REPORT

**Application Number**

EP 21 21 7220

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/069954 A1 (COHEN BENJAMIN [IL] ET AL) 7 March 2019 (2019-03-07)<br>* paragraph [0008] – paragraph [0017] *<br>* paragraph [0049] – paragraph [0066]; figures 5,6 * | 1-15 | INV.<br>G06T17/20 |
| A | US 2018/158238 A1 (COHEN ASSAF [IL] ET AL) 7 June 2018 (2018-06-07)<br>* paragraph [0024] – paragraph [0025]; figure 1 *<br>* paragraph [0033] – paragraph [0048]; figures 2A-C, 3 * | 1-15 | |
| A | ROMANOV ALEXANDER ET AL: "High-resolution, real-time, and nonfluoroscopic 3-dimensional cardiac imaging and catheter navigation in humans using a novel dielectric-based system", HEART RHYTHM, ELSEVIER, US, vol. 16, no. 12, 27 June 2019 (2019-06-27), pages 1883-1889, XP085925327, ISSN: 1547-5271, DOI: 10.1016/J.HRTHM.2019.06.020 [retrieved on 2019-06-27]<br>* page 1, column 1, line 15 – page 2, column 2, line 27 * | 1-15 | TECHNICAL FIELDS SEARCHED (IPC)<br><br>G06T |

The present search report has been drawn up for all claims

| Place of search | Date of completion of the search | Examiner |
|---|---|---|
| The Hague | 13 June 2022 | Martos Riaño, Demian |

## ANNEX TO THE EUROPEAN SEARCH REPORT
## ON EUROPEAN PATENT APPLICATION NO.

EP 21 21 7220

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2022

| Patent document cited in search report | | Publication date | Patent family member(s) | | Publication date |
|---|---|---|---|---|---|
| US 2019069954 | A1 | 07-03-2019 | AU 2018220134 | A1 | 21-03-2019 |
| | | | BR 102018067920 | A2 | 07-05-2019 |
| | | | CA 3015087 | A1 | 06-03-2019 |
| | | | CN 109452948 | A | 12-03-2019 |
| | | | EP 3453328 | A1 | 13-03-2019 |
| | | | IL 261386 | A | 29-04-2021 |
| | | | JP 2019042514 | A | 22-03-2019 |
| | | | KR 20190027326 | A | 14-03-2019 |
| | | | RU 2018130839 | A | 28-02-2020 |
| | | | US 2019069954 | A1 | 07-03-2019 |
| US 2018158238 | A1 | 07-06-2018 | AU 2017258897 | A1 | 21-06-2018 |
| | | | CA 2987535 | A1 | 06-06-2018 |
| | | | CN 108171779 | A | 15-06-2018 |
| | | | EP 3333807 | A1 | 13-06-2018 |
| | | | IL 255529 | A | 26-09-2019 |
| | | | JP 7066387 | B2 | 13-05-2022 |
| | | | JP 2018089381 | A | 14-06-2018 |
| | | | MA 45306 | A | 13-06-2018 |
| | | | US 2018158238 | A1 | 07-06-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017201288 A1
- US 2021052191 A1
- WO 2012150567 A1

- EP 0775466 A2
- EP 3568068 A1
- EP 3607879 A1

**Non-patent literature cited in the description**

- **BERGER, MATTHEW et al.** A survey of surface reconstruction from point clouds. *Computer Graphics Forum,* 2017, vol. 26 (1